# EUROPEAN PATENT SPECIFICATION

(11) **EP 3 787 544 B1**
(45) Date of publication and mention of the grant of the patent: **08.06.2022**
(21) Application number: 19727915.1
(22) Date of filing: 30.04.2019
(51) Int. Cl.: A61C 3/04, A61C 1/08

(54) **CONTAINER FOR DEPTH STOP FOR DENTAL DRILLS**
BEHÄLTER FÜR TIEFENANSCHLAG FÜR DENTALBOHRER
RÉCIPIENT POUR BUTÉE DE PROFONDEUR POUR FORETS DENTAIRES

(30) Priority: 03.05.2018 IT 201800005038
(43) Date of publication of application: 10.03.2021
(73) Proprietor: Leone S.p.A., 50019 Sesto Fiorentino (Firenze) (IT)
(72) Inventor: SCOMMEGNA, Gabriele, 50029 Tavernuzze Impruneta (FI) (IT); DOLFI, Maurizio, 50143 Firenze (IT)
(74) Representative: Mincone, Antimo
(86) International application number: PCT/IT2019/050087
(87) International publication number: WO 2019/211882

(56) References cited:
- EP-A1- 1 867 297
- EP-A2- 2 433 587
- CN-A- 106 691 611

## Description

The present invention relates to a container for depth stops for dental burs.

Depth stops for dental burs are devices designed to adjust the depth of the drilling depth of dental burs or drills. The depth stops consist of a substantially cylindrical hollow body that can be fitted at the distal end of the dental burs and allows only a part of the dental bur to remain free so as to determine a limit stop, i.e. to stop the osteotomy at a predetermined depth value when the depth stop comes into contact with the surface interested by the osteotomy.

The depth stops are shaped and sized according to the dental bur to be equipped and the desired depth value.

During use, when the doctor has to work with the dental bur provided with depth stop, he chooses a depth stop having a diameter suitable for the dental bur used and having a height corresponding to the value of the chosen working depth and then he puts it on the dental bur, binding it to the latter thanks to a locking element provided on the depth stop and able to engage a corresponding seat provided on the dental bur body.

At the end of the surgical procedure or of a phase of the same, it is necessary to disassociate the depth stop from the dental bur; this operation is not always easy and can cause problems for the doctor, for example due to loss of time, possible damage to the dental burs and for the management of the surgical procedure in general.

EP1867297 describes a kit that includes a plurality of depth stops of different diameter made of transparent plastic material and contained in a box in which they are stored packed in blisters. EP1867297 also shows a block for mounting depth stops on the dental burs and for the subsequent disassembly. The block is provided with a series of through holes which have different diameters; for the mounting of the depth stops the doctor must put a depth stop in the seat of the corresponding diameter and then insert the dental bur in the depth stop so positioned so as to associate the same depth stop with the dental bur; to disassemble the depth stop from the dental bur, the doctor must insert the dental bur in another hole, provided with a reduced diameter portion through which passes only the dental bur so as to detach the stop from the same dental bur. The EP1867297 block is used only in the operations of assembly and disassembly of the stops and the holes that are provided in it do not allow the doctor to recognize the depth of the stop during assembly. In practice, the holes used for mounting the stops (as well as those used for disassembly) are differentiated only according to the diameter of the dental bur to be equipped; in this way the mounting block cannot give any indication to the doctor about the depth of the stop that is to be mounted on the dental bur.

In other words, the mounting block also allows to mount on the dental bur a depth stop that does not correspond to that of the procedure to be performed on the patient. It is evident that the use of an incorrect depth stop can cause serious damage to the patient. An aim of the present invention is to provide a new container for depth stops capable of eliminating the mentioned drawbacks and to provide a method for mounting the depth stop on the dental bur and for disassociating the stop from the dental bur able to guarantee a high degree of safety combined with low costs if compared to the quality of the product.

This result is achieved in accordance with the invention by adopting the idea of creating a container for depth stops having the features described in claim 1. Other features are described in the dependent claims.

Among the advantages of the present invention it is possible to list the following: the container of the invention allows to house a plurality of depth stops for dental burs and to fit the depth stop on a dental bur and extract the stop fitted on a dental bur with extreme ease, guaranteeing a unique recognition during the assembly of the diameter and the depth of the stop; the container of the invention is of simple construction and does not involve an excessive increase of resources in its realization if compared to a container made of the same material and to the advantages provided; the container can be provided with an element to facilitate the disassembly of the stop from the dental bur, which further increases safety for the operators and reduces the possibility of damaging the dental bur or other objects; the use of the container is remarkably intuitive, so making easy to understand the correct functioning of the same container-extractor; the innovative features are maintained even with prolonged use of the container; production costs are not higher than those of known techniques, although they offer a higher quality product.

These and further advantages and features of the present invention will be more and better understood by every person skilled in the art from the following description and with the aid of the attached drawings, given as a practical exemplification of the invention, but not to be considered in a limiting sense, in which:

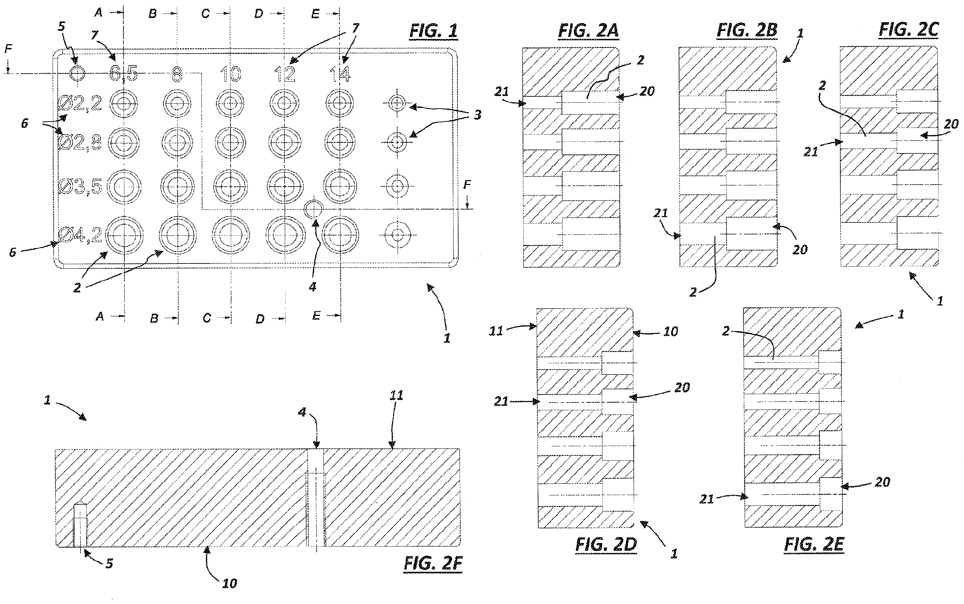

- Fig. 1 is a top plan view of a possible embodiment of a container for depth stops for dental burs in accordance with the invention;

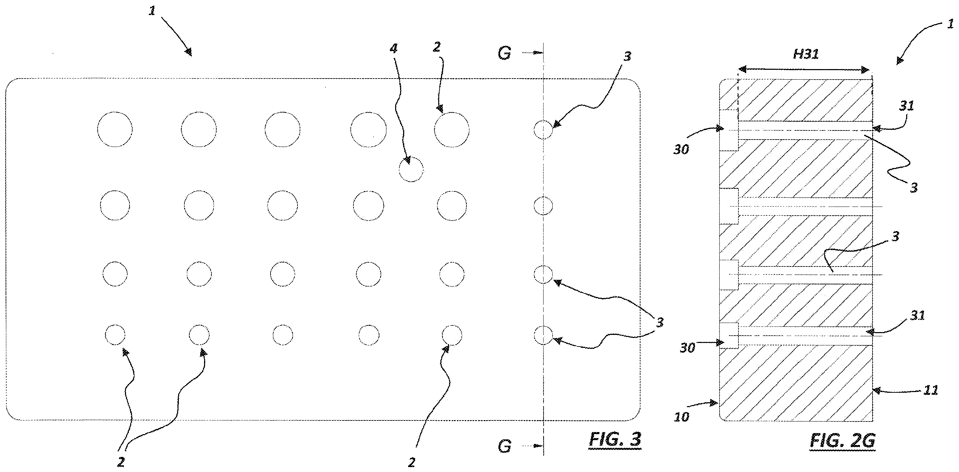

- Figs. 2A, 2B, 2C, 2D, 2E 2F, 2G are sectional views of the example shown in Fig.1 and Fig.3 obtained, respectively, by means of the section lines AA (Fig.2A), BB (Fig.2B ), CC (Fig.2C), DD (Fig.2D), EE (Fig.2E), FF (Fig.2F) of Fig.1 and by means of the GG line of Fig.3 (Fig.2G);
- Fig. 3 shows the example of Fig. 1 in a plan view from below;
- Figs. 4, 5, 6 show a possible embodiment example of the container for depth stops provided with a cover and represented, respectively, in a perspective view (Fig.4), in a top plan view (Fig.5), and in a sectional view according to the section line VI-VI of Fig. 5 (Fig. 6);
- Figs. 7, 8, 9 are schematic side views showing a possible example of a dental bur (Fig. 7), a depth stop (Fig. 8) and the association of a depth stop to a dental bur (Fig. 9);
- Figs. 10A, 10B, 10C show, by means of schematic perspective views, possible steps of the procedure for associating the depth stop with the dental bur;
- Figs. 11A, 11B, 11C show, by means of schematic perspective views, possible steps of the procedure for disassociating the depth stop from the dental bur;

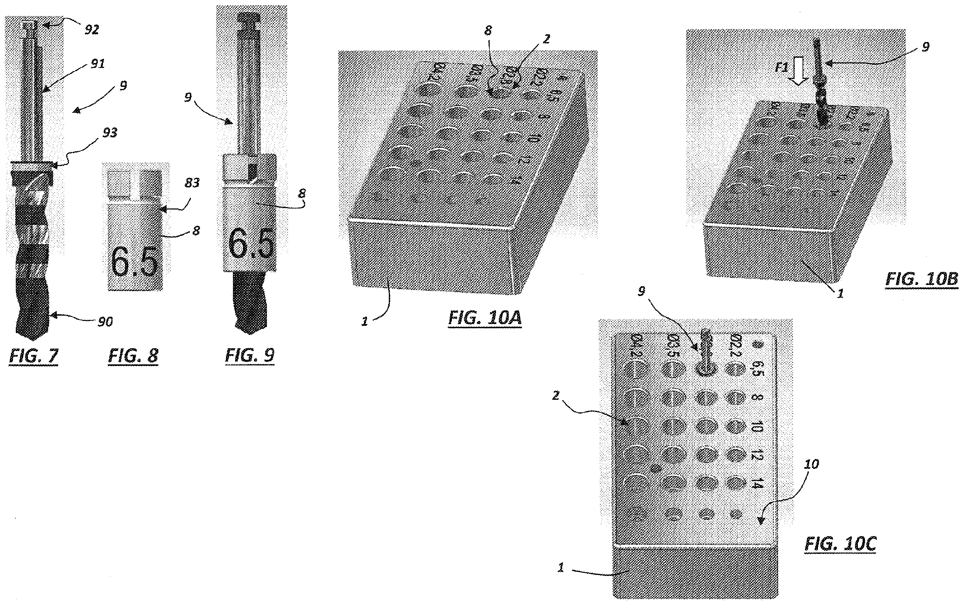

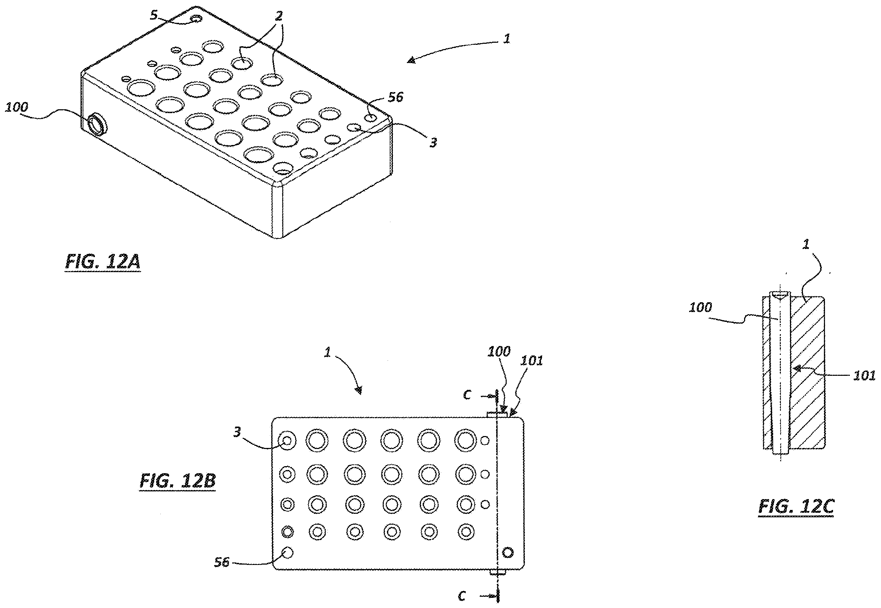

- Figs. 12A, 12B and 12C represent a further possible embodiment of the container which in this case is provided with an element to facilitate disassembly of the depth stop from the dental bur; Fig. 12A is a perspective view, Fig. 12B is a view from above and Fig. 12C is a section view along the line C-C of Fig. 12B. Figs. 7-9 show possible embodiment of a dental bur (9) of a depth stop (8) and of the two associated elements. The dental bur (9), in the view of Fig.7, comprises: a proximal portion (92) shaped so as to allow the association with a dental handpiece (not shown), a stem (91) and a tip (90). Between the tip (90) and the stem (91) there is a median portion (93) shaped so as to be coupled with a depth stop (8) fitted on the dental bur (9). The stop (8) shown in Figs. 8 and 9 is marked with the value "6.5" i.e. with the depth value that the stop determines when it is mounted on the dental bur (9). As previously indicated, the depth stops (8) are differentiated according to the diameter of the dental bur (9) to be equipped and the drilling depth they determine.

For this purpose the container (1) shown in the other attached figures is provided with a plurality of seats (2), arranged according to two series of values to identify the depth stops (8) housed therein. In practice, referring to the non-limiting values of the drawings, a first value (6) indicates the diameter (2,2 - 2.,8 - 3,5 - 4,2) and a second value (7) indicates the depth (6,5 - 8 - 10 - 12 - 14). Each of the seats (2) has two cylindrical portions with different diameters. The cylindrical portion (20) facing upwards, i.e. open on the upper face (10) of the container (1), has a larger diameter; the cylindrical portion (21) facing downwards, i.e. open on the lower face (11) of the container (1), has a smaller diameter. The larger diameter portion (20) of the seat (2) is shaped and sized so as to accommodate the depth stop (8) while the smaller diameter portion (21) is shaped and sized so as to accommodate the tip (90) of the corresponding dental bur (9) on which to fit the depth stop (8) during the phase of association between the two elements (8, 9).

In practice, the container (1) has a first plurality of through seats (2) for the depth stops (8), arranged according to a matrix in which the seats are differentiated by the value (6) of the diameter in their arrangement along the rows and by the depth value (7) in the arrangement along the columns.

The container (1) can be made of titanium or other convenient material; the values written (6) and (7) can be made by laser marking or other suitable means for this purpose.

Moreover, said written values (6) and (7) can be made in colors that are different from each other and/or can be combined with symbols applied to the container and made in different colors. The same colors used for the values (6) and (7) can be used for the corresponding tips of the dental burs (9) and for the depth stops (8). In this way the combination between depth stop (8) and tip (9) is easier and safer, creating a sort of "color code" to identify more easily the combinations to be defined. For this purpose the middle portion (93) of the dental bur can be provided with a colored ring and the stop (8) can also be provided with a colored ring (83).

The container (1) is provided with a second plurality of seats (3) which in the illustrated example are arranged on the right side of the container (1), to the right of the matrix formed by the first plurality of seats (2).

Each of the seats (3) has two cylindrical parts with different diameters. The part (30) facing upwards, i.e. towards the upper face (10) of the container (1) has a larger diameter; the part (31) facing downwards, i.e. towards the lower face (11) of the container (1) has a smaller diameter. The larger diameter part (30) of the seat (3) is shaped and sized so as to accommodate the depth stop (8) while the smaller diameter part (31) is shaped and sized so as to accommodate the stem (91) of the corresponding dental bur (9) on which the depth stop is fitted (8). As will be better described below, the seats (3) of the second plurality are used in the phase of disassociation between the two elements (8) and (9).

The seats (3) shown in the example of the drawings are through holes and arranged along a column, differentiated on the basis of the value (6) of the depth stop (8) to be received.

Figs. 10A-C schematically show the procedure for associating the depth stop (8) with the dental bur (9). A depth stop (8) is placed in the corresponding seat (2) of the container (1) at the larger diameter section (20). The dental bur (9), with the tip (90) facing downwards, is introduced into the depth stop (8) with the movement indicated by the arrow (F1) in Fig. 10B, exerting a downward thrust that determines the fixing of the depth stop (8) to the dental bur (9), thanks to the connection of the same depth stop (8) with the middle portion (93) of the dental bur (9). In the downward movement the tip (90) passes through the smaller portion (21) of the seat (2).

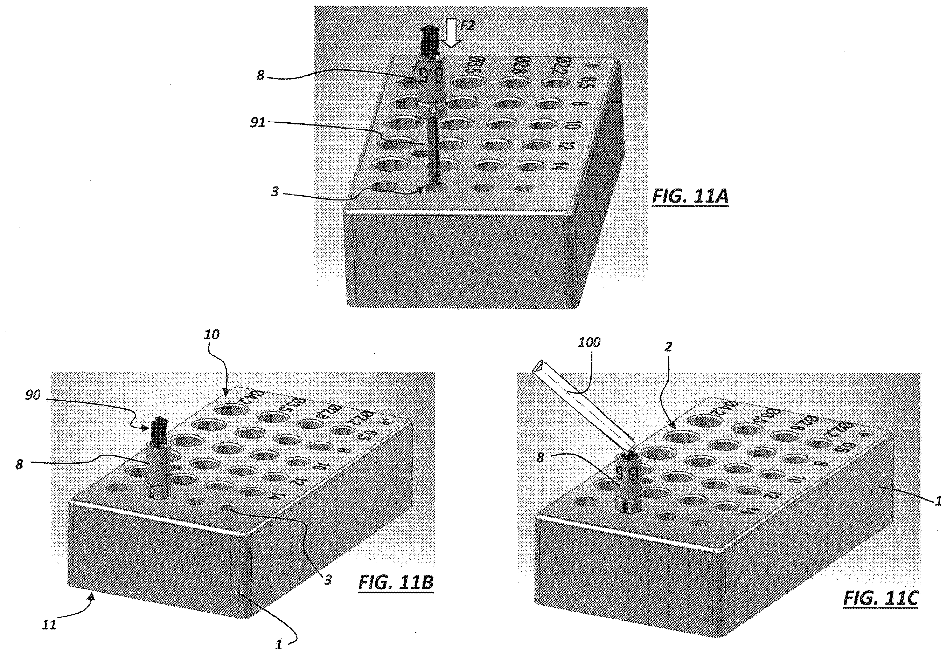

Figs. 11A-C schematically show the procedure for detaching the depth stop (8) from the dental bur (9). A dental bur (9) fitted with a depth stop (8) is introduced into the seat (3), from the part with the largest section (30), with the stem (91) facing downwards, with the movement indicated by the arrow (F2) in Fig 11A. The insertion of the assembly formed by dental bur (9) and depth stop (8) in the seat (3) reaches a limit stop when the stop (8) is received in the larger diameter portion (30) of the seat (3). The subsequent downward movement of the dental bur (9) causes the dental bur (9) to be released from the depth stop (8). The depth or height (H31) of the smaller diameter part (31) may be greater than the length of the dental bur (9) so as to allow the entire extraction of the dental bur with respect to the stop (8) fitted on it; in other words, when the container (1) rests on a surface, the dental bur (9) does not come out of the lower face (11) and does not interfere with the same surface that could prevent it from slipping off.

In this way, the container (1) allows to associate and disassociate the dental bur (9) from the relative depth stop (8) in a simple, effective and safe way.

The safety in choosing the depth stop (8) to be mounted on the dental bur (9) is therefore guaranteed, both in terms of diameter and depth. In fact, it is the same container (1) that constitutes the unit for assembling the depth stops on the dental burs and which determines a unique correspondence between the choice of the depth stop and the corresponding assembly on the dental bur.

On the container (1) there is a seat (5) and a through hole (4) that connects the upper face (10) to the lower face (11).

The seat (5) houses a pin (52) which, passing through a corresponding seat (53), constrains a cover (50) to the container (1). In practice, the cover (50) has a plan conformation corresponding to that of the container (1) and can rotate around the axis defined by the pin (52) to pass from a closed position in which it is superimposed on the upper face (10 ) of the container (1) to an open position in which it leaves the said upper face (10) uncovered and allows access to the seats (2) and (3) placed there. Advantageously, the cover (50) is provided with a plurality of through holes (51) arranged in correspondence of the seats (2) and (3) presented by the container (1). In this way it is possible to convey fluids (for example during sterilization operations) through the seats (2) and (3) even with the cover (50) closed.

The container can be provided with a locking element (56) which protrudes from the upper surface of the container to interfere with the lower face of the cover (50) when the latter is in the closed configuration. The locking element (56) can have, for example, a spherical conformation.

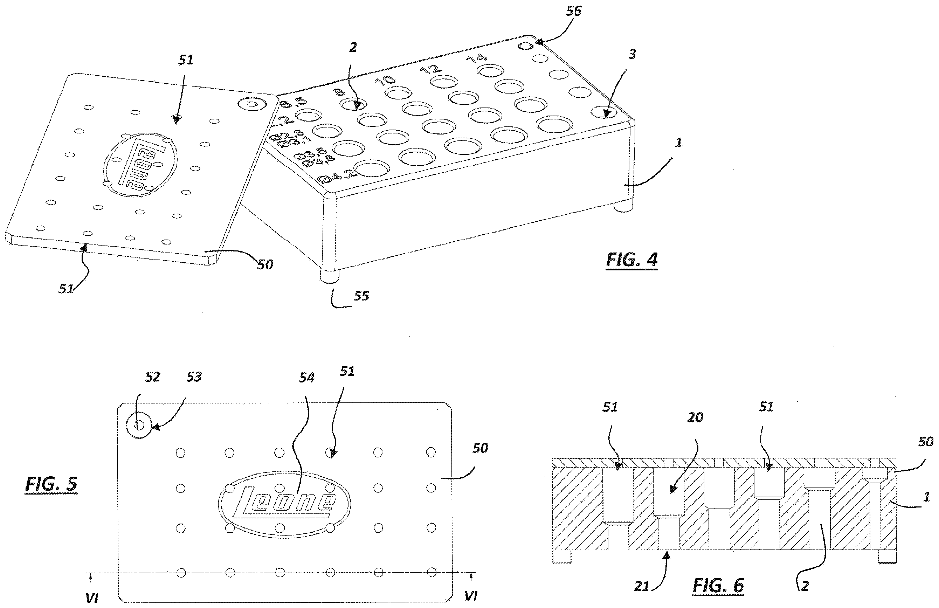

Fig. 4 shows an open configuration of the container (1).

The cover (50) can be written and/or marked (54) according to a per se known manner. Furthermore, the container (1) can be provided with supporting feet (55) such as those shown in Figs.4-6.

Figs. 12A-C show a possible embodiment of the container (1) which is provided with an element (100) to facilitate disassembly of the stop from the dental bur. In practice, when the dental bur (9) is inserted in one of the holes (3) of the container (1) (as schematically shown in Fig. 11C), the element to facilitate disassembly (100) can be used by the doctor for push the tip (90) downwards without using directly his fingers, so increasing safety. The element (100) can have a cylindrical or partially frusto-conical shape and can be made of a suitable plastic material so as not to damage the tip (90) of the dental bur (9). The container (1) can be provided with a corresponding seat (101) for stably accommodating the element (100) when it is not used.

In accordance with the present invention, a container (1) for depth stops (8) for dental burs adapted to be mounted on a dental bur (9) is of the type that comprises a first plurality of seats (2) for housing the depth stops (8) with different diameters and / or heights; the container (1) is characterized in that it has at least one further seat (3) having a part (31) of smaller diameter than any one of said seats (2), said at least one further seat (3) allowing the passage of a part (91) of a dental bur (9) so as to remove the depth stop (8) from the dental bur (9) on which it is mounted, and in that said first plurality of seats (2) is arranged according to two series of values, the first value (6) indicating the diameter of the depth stop (8) and the second value (7) indicating the height of the depth stop (8).

In accordance with what is described and illustrated in the drawings, said at least one further seat (3), in addition to said smaller diameter part (31), also has a larger diameter part (30) connected to the smaller diameter part.

The part (31) with a smaller diameter is substantially a through hole having a smaller diameter with respect to another through hole with a larger diameter which forms said part (30) with a larger diameter.

Moreover, said at least one further seat (3) has a height (H31) greater than the length of said part (91) of the dental bur (9).

Still with reference to what has been described and illustrated in the drawings, said at least one seat (3) is comprised in a second plurality of seats (3) having the respective sections with a larger diameter (30) differentiated according to the stop (8) to be received. Said first value (6) and / or said second value (7) are provided with a colored identification index which corresponds to the corresponding color code presented by a tip (90) and / or a stop (8) to be associated with the dental bur ( 9). Moreover, each of the seats (2) of said first plurality has two cylindrical portions with different diameters; the larger diameter portion (20) being shaped and sized so as to accommodate a stop (8), the smaller diameter portion (21) being shaped and sized so as to accommodate the tip (90) of the corresponding dental bur (9) on which to put the stop (8).

Still with reference to what has been described and illustrated in the drawings, said part of smaller diameter (31) is shaped and sized so as to accommodate the stem (91) of a dental bur (9) from which the relative stop (8) can be extracted.

Still with reference to what has been described and illustrated in the drawings, the container (1) is provided with a closure cover (50). Moreover, the cover (50) is provided with a plurality of holes (51) arranged at a corresponding plurality of seats (2, 3) presented by the container (1).

Still with reference to what has been described and illustrated in the drawings, the container (1) comprises an element (100) to facilitate disassembly of the depth stop (8) from the dental bur (9): the container (1) is provided with a seat (101) for the stable containment of said element (100).

The invention also relates to a depth stop kit (8) for dental burs adapted to be fitted on a dental bur (9), the container comprising a first plurality of seats (2) for housing the depth stops (8), having different diameters and/or heights, the kit comprises a container (1) according to what previously described and illustrated in the drawings and a plurality of depth stops inserted in said seats (2).

The details of execution can however vary in an equivalent manner in the shape, dimensions, arrangement of the elements, nature of the materials used, without however departing from the scope of the idea of solution adopted and therefore remaining within the limits of the protection granted by the present patent.

## Claims

1. Container (1) for depth stops (8) for dental burs suitable for being mounted on a dental bur (9), the container comprising a first plurality of seats (2) for housing the depth stops (8) having different diameters and/or heights, wherein it has at least one further seat (3) having a part (31) of smaller diameter than any one of said seats of the first plurality of seats (2), said at least one further seat (3) allowing the passage of a part (91) of a dental bur (9) so as to remove the depth stop (8) from the dental bur (9) on which it is mounted, and in that said first plurality of seats (2) is arranged according to two series of values, the first value (6) indicating the diameter of the depth stop (8) and the second value (7) indicating the height of the depth stop (8), said portion of smaller diameter (31) of the at least one further seat (3) being shaped and sized so as to accommodate the stem (91) of a dental bur (9) from which the relative stop is to be extracted (8), said at least one further seat (3), in addition to said smaller diameter part (31), also having a larger diameter part (30) connected to the smaller diameter part, each of the seats (2) of said first plurality of seats having two cylindrical portions with different diameters (20, 21), with the larger diameter portion (20) being shaped and sized so as to accommodate a stop (8), and the smaller diameter portion (21) being shaped and sized so as to accommodate the tip (90) of the corresponding dental bur (9) on which to fit the stop (8).

2. Container (1) according to claim 1, **characterized in that** said at least one further seat (3) has a height (H31) greater than the length of said part (91) of the dental bur (9).

3. Container (1) according to claim 1, **characterized in that** said at least one seat (3) is comprised in a second plurality of seats (3) having the respective sections with a larger diameter (30) differentiated according to the stop (8) to be received.

4. Container (1) according to claim 1, **characterized in that** said first value (6) and / or said second value (7) are provided with a colored identification index which corresponds to the corresponding color code presented by a dental bur (9) and by a stop (8) to be associated with the dental bur (9).

5. Container (1) according to one of the preceding claims, **characterized in that** it is provided with a closure cover (50).

6. Container (1) according claim 5, **characterized in that** said cover (50) is provided with a plurality of holes (51) arranged at a corresponding plurality of seats (2, 3) presented by the container (1).

7. Container (1) according to one of the preceding claims, **characterized in that** it comprises an element (100) to facilitate the disassembly of the depth stop (8) from the dental bur (9).

8. Container (1) according to claim 7, **characterized in that**, said container (1) is provided with a seat (101) for the stable containment of said element (100).

9. Depth stop kit (8) for dental burs, suitable for being mounted on a dental bur (9), the container comprising a first plurality of seats (2) for housing the depth stops (8), having different diameters and/or heights, kit **characterized in that** it comprises a container (1) according to one or more of the preceding claims and a plurality of depth stops (8) inserted in said seats (2).

## Patentansprüche

1. Behälter (1) für Tiefenstopps (8) für Zahnbohrer, der dazu geeignet ist, auf einem Zahnbohrer (9) montiert zu werden, wobei der Behälter eine erste Vielzahl von Sitzen (2) zur Aufnahme der Tiefenstopps (8) mit unterschiedlichen Durchmessern und/oder Höhen umfasst, wobei er mindestens einen weiteren Sitz (3) mit einem Teil (31) mit kleinerem Durchmesser als jeder der Sitze der ersten Vielzahl von Sitzen (2) aufweist, der mindestens eine weitere Sitz (3) den Durchgang eines Teils (91) eines Zahnbohrers (9) ermöglicht, um den Tiefenstopp (8) von dem Zahnbohrer (9) zu entfernen, auf dem er montiert ist, und dass die erste Vielzahl von Sitzen (2) gemäß zwei Reihen von Werten angeordnet ist, wobei der erste Wert (6) den Durchmesser des Tiefenstopps (8) angibt und der zweite Wert (7) die Höhe des Tiefenstopps (8) angibt, wobei der Abschnitt mit kleinerem Durchmesser (31) des mindestens einen weiteren Sitzes (3) so geformt und bemessen ist, dass er den Schaft (91) eines Zahnbohrers (9) aufnimmt, aus dem der entsprechende Stopp (8) herausgezogen werden soll, wobei der mindestens eine weitere Sitz (3) zusätzlich zu dem Teil mit kleinerem Durchmesser (31) auch einen Teil mit größerem Durchmesser (30) aufweist, der mit dem Teil mit kleinerem Durchmesser verbunden ist, wobei jeder der Sitze (2) der ersten Vielzahl von Sitzen zwei zylindrische Abschnitte mit unterschiedlichen Durchmessern (20, 21) aufweist, wobei der Abschnitt (20) mit dem größeren Durchmesser so geformt und bemessen ist, dass er einen Stopp (8) aufnimmt, und der Abschnitt (21) mit dem kleineren Durchmesser so geformt und bemessen ist, dass er die Spitze (90) des entsprechenden Zahnbohrers (9) aufnimmt, auf den der Stopp (8) aufgesetzt wird.

2. Behälter (1) nach Anspruch 1, **dadurch gekennzeichnet, dass** der mindestens eine weitere Sitz (3) eine Höhe (H31) aufweist, die größer als die Länge des Teils (91) des Zahnbohrers (9) ist.

3. Behälter (1) nach Anspruch 1, **dadurch gekennzeichnet, dass** der mindestens eine Sitz (3) in einer zweiten Vielzahl von Sitzen (3) umfasst ist, deren jeweilige Abschnitte einen größeren Durchmesser (30) aufweisen, der sich nach dem aufzunehmenden Stopp (8) unterscheidet.

4. Behälter (1) nach Anspruch 1, **dadurch gekennzeichnet, dass** der erste Wert (6) und/oder der zweite Wert (7) mit einem farbigen Identifikationsindex bereitgestellt sind, der dem entsprechenden Farbcode entspricht, der von einem Zahnbohrer (9) und von einem dem Zahnbohrer (9) zuzuordnenden Stopp (8) dargestellt wird.

5. Behälter (1) nach einem der vorstehenden Ansprüche, **dadurch gekennzeichnet, dass** er mit einem Verschlussdeckel (50) bereitgestellt ist.

6. Behälter (1) nach Anspruch 5, **dadurch gekennzeichnet, dass** der Deckel (50) mit einer Vielzahl von Löchern (51) bereitgestellt ist, die an einer entsprechenden Vielzahl von Sitzen (2, 3) angeordnet sind, die der Behälter (1) präsentiert.

7. Behälter (1) nach einem der vorstehenden Ansprüche, **dadurch gekennzeichnet, dass** er ein Element (100) umfasst, das die Demontage des Tiefenstopps (8) vom Zahnbohrer (9) erleichtert.

8. Behälter (1) nach Anspruch 7, **dadurch gekennzeichnet, dass** der Behälter (1) mit einem Sitz (101) für die stabile Aufnahme des Elements (100) bereitgestellt ist.

9. Tiefenstopp-Set (8) für Zahnbohrer, das dazu geeignet ist, auf einem Zahnbohrer (9) montiert zu werden, wobei der Behälter eine erste Vielzahl von Sitzen (2) zur Aufnahme der Tiefenstopps (8) umfasst, die unterschiedliche Durchmesser und/oder Höhen aufweisen, wobei das Set **dadurch gekennzeichnet ist, dass** es einen Behälter (1) nach einem oder mehreren der vorstehenden Ansprüche und eine Vielzahl von Tiefenstopps (8) umfasst, die in die Sitze (2) eingesetzt sind.

## Revendications

1. Récipient (1) pour butées de profondeur (8) pour des butées de profondeur appropriées pour être montées sur une fraise dentaire (9), le récipient comprenant une première pluralité de sièges (2) pour loger les butées de profondeur (8) présentant différents diamètres et/ou hauteurs, dans lequel il présente au moins un autre siège (3) présentant une partie (31) d'un diamètre plus petit que l'un quelconque desdits sièges de la première pluralité de sièges (2), ledit au moins un autre siège (3) permettant le passage d'une partie (91) d'une fraise dentaire (9) de façon à retirer la butée de profondeur (8) de la fraise dentaire (9) sur laquelle elle est montée, et en ce que ladite première pluralité de sièges (2) est agencée selon deux séries de valeurs, la première valeur (6) indiquant le diamètre de la butée de profondeur (8) et la seconde valeur (7) indiquant la hauteur de la butée de profondeur (8), ladite partie de diamètre plus petit (31) du au moins un autre siège (3) étant formée et dimensionnée de façon à loger la tige (91) d'une fraise dentaire (9) de laquelle la butée relative doit être extraite (8), ledit au moins un autre siège (3), en plus de ladite partie de diamètre plus petit (31), présentant également une partie de diamètre plus grand (30) connectée à la partie de diamètre plus petit, chacun des sièges (2) de ladite pluralité de sièges présentant deux parties cylindriques avec différents diamètres (20, 21), la partie de diamètre plus grand (20) étant formée et dimensionnée de façon à loger une butée (8), et la partie de diamètre plus petit (21) étant formée et dimensionnnée de façon à loger la pointe (90) de la fraise dentaire correspondante (9) sur laquelle adapter la butée (8).

2. Récipient (1) selon la revendication 1, **caractérisé en ce que** ledit au moins un autre siège (3) présente une hauteur (H31) supérieure à la longueur de ladite partie (91) de la fraise dentaire (9).

3. Récipient (1) selon la revendication 1, **caractérisé en ce que** ledit au moins un siège (3) est compris dans une seconde pluralité de sièges (3) présentant les sections respectives avec un diamètre plus grand (30) différenciées en fonction de la butée (8) à recevoir.

4. Récipient (1) selon la revendication 1, **caractérisé en ce que** ladite première valeur (6) et/ou ladite seconde valeur (7) sont dotées d'un indice d'identification de couleur qui correspond au code couleur correspondant présenté par une fraise dentaire (9) et par une butée (8) à associer à la fraise dentaire (9).

5. Récipient (1) selon l'une quelconque des revendications précédentes, **caractérisé en ce qu'**il est doté d'un couvercle de fermeture (50).

6. Récipient (1) selon la revendication 5, **caractérisé en ce que** ledit couvercle (50) est doté d'une pluralité de trous (51) agencés au niveau d'une pluralité de sièges correspondants (2, 3) présentés par le récipient (1).

7. Récipient (1) selon l'une quelconque des revendications précédentes, **caractérisé en ce qu'**il comprend un élément (100) pour faciliter le démontage de la butée de profondeur (8) depuis la fraise dentaire (9).

8. Récipient (1) selon la revendication 7, **caractérisé en ce que** ledit récipient (1) est doté d'un siège (101) pour le confinement stable dudit élément (100).

9. Kit de butées de profondeur (8) pour fraises dentaires, appropriées pour être montées sur la fraise dentaire (9), le récipient comprenant une première pluralité de sièges (2) pour loger les butées de profondeur (8), présentant différents diamètres et/ou hauteurs, le kit étant **caractérisé en ce qu'**il comprend un récipient (1) selon une ou plusieurs des revendications précédentes et une pluralité de butées de profondeur (8) insérées dans lesdits sièges (2).
